# FASCICULE DE BREVET EUROPEEN

(11) **EP 3 624 807 B1**
(45) Date de publication et mention de la délivrance du brevet: **07.07.2021**
(21) Numéro de dépôt: 18723038.8
(22) Date de dépôt: 16.05.2018
(51) Int. Cl.: A61K 31/70, A61P 17/02

(54) **UTILISATION DE COMPOSES OLIGOSACCHARIDIQUES POUR TRAITER LES PLAIES DES PATIENTS DIABETIQUES ARTERIOPATIQUES**
VERWENDUNG VON OLIGOSACCHARIDVERBINDUNGEN ZUR BEHANDLUNG VON WUNDEN BEI DIABETESPATIENTEN MIT ARTERIOPATHIEN
USE OF OLIGOSACCHARIDE COMPOUNDS FOR TREATING WOUNDS OF ARTERIOPATHIC DIABETIC PATIENTS

(30) Priorité: 17.05.2017 FR 1754363; 20.06.2017 FR 1755620
(43) Date de publication de la demande: 25.03.2020
(73) Titulaire: Urgo Recherche Innovation et Développement, 21300 Chenove (FR)
(72) Inventeur: BOHBOT, Serge, 92300 Levallois-Perret (FR)
(74) Mandataire: Plasseraud IP
(86) Numéro de dépôt international: PCT/EP2018/062811
(87) Numéro de publication internationale: WO 2018/210969

(56) Documents cités:
- WO-A1-03/035656
- WO-A1-2014/020031
- WO-A2-2014/009488
- WO-A2-2015/177379
- YOTSU RIE ROSELYNE ET AL: "Comparison of characteristics and healing course of diabetic foot ulcers by etiological classification: Neuropathic, ischemic, and neuro-ischemic type", JOURNAL OF DIABETES AND ITS COMPLICATIONS, vol. 28, no. 4, juillet 2014 (2014-07), pages 528-535, XP002778939,
- AKARSH Y.G. & NISCHAL K.: "A comparative study of topical human placental extract with topical sucralfate in the management of diabetic foot ulcers", INTERNATIONAL JOURNAL OF CURRENT RESEARCH, vol. 8, no. 03, mars 2016 (2016-03), pages 28501-28503, XP002778940,

## Description

La présente invention a pour objet un oligosaccharide polysulfaté synthétique ayant 1 à 4 unités oses, ses sels, ou ses complexes, pour son utilisation pour traiter l'ulcère du pied du diabétique chez des patients artériopathiques, en particulier pour activer la cicatrisation de l'ulcère du pied du diabétique chez des patients artériopathiques.

La cicatrisation d'une plaie est un phénomène biologique naturel, les tissus mammifères étant capables de réparer des lésions localisées par des processus de réparation et de régénération qui leur sont propres.

La rapidité et la qualité de la cicatrisation d'une plaie dépendent de l'état général de l'organisme atteint, de l'étiologie de la plaie, de l'état et de la localisation de la plaie, et de la survenue ou non d'une infection, ainsi que des facteurs génétiques prédisposant ou non à des troubles de la cicatrisation.

La cicatrisation naturelle d'une plaie se déroule principalement selon trois phases successives, chacune de ces phases étant caractérisée par des activités cellulaires spécifiques qui font progresser le processus de réparation selon des séquences chronologiques précises : la phase inflammatoire, la phase de granulation (ou phase proliférative), et la phase de maturation.

La première phase, la phase inflammatoire, débute dès la rupture des vaisseaux sanguins qui déclenche la formation d'un caillot (coagulation du sang) principalement composé de fibrine et de fibronectine, et qui va constituer une matrice provisoire. Cette matrice comble en partie la lésion et va permettre la migration au sein de la zone lésée des cellules inflammatoires recrutées pour assurer la détersion de la plaie. Les plaquettes présentes vont également libérer des facteurs (par exemple cytokine, facteurs de croissance) permettant le recrutement des cellules de la cicatrisation comme les cellules inflammatoires (les polynucléaires neutrophiles et les macrophages), les fibroblastes et les cellules endothéliales.

La seconde phase correspond au développement du tissu de granulation. On observe d'abord une colonisation de la blessure par prolifération des fibroblastes. Puis, la migration des cellules endothéliales à partir des vaisseaux sains va permettre la formation de nouveaux vaisseaux sanguins (néovascularisation), ou angiogénèse, du tissu lésé. Cette étape d'angiogenèse est fondamentale pour amorcer la cicatrisation. Dans le tissu de granulation, les fibroblastes sont activés et vont se différencier en myofibroblastes présentant des propriétés contractiles importantes, générées par les microfilaments d'actine, permettant la contraction de la plaie.

La troisième phase du processus de réparation, la maturation, s'accompagne d'un remodelage du tissu de granulation. Une partie de la matrice extracellulaire est digérée par des protéases (essentiellement des métallo-protéases matricielles (MMP) et des élastases), et on observe une réorganisation progressive de la matrice extracellulaire. Progressivement, le collagène de type III, majoritaire dans le tissu de granulation, est remplacé par le collagène de type I, principal composant matriciel du derme. A la fin de la phase de maturation, les fibroblastes, myofibroblastes et cellules vasculaires voient leur prolifération et/ou leur activité réduites. Puis les cellules excédentaires meurent par apoptose. Parallèlement au remodelage de la matrice extracellulaire et à l'apoptose des cellules excédentaires, l'état inflammatoire diminue progressivement. Cette phase est la plus longue : au bout d'un an environ, la cicatrice se remodèle, elle n'est plus rouge, ni rigide, ne provoque plus de douleur et elle s'aplanie.

Néanmoins, certains types de plaies ne cicatrisent pas correctement, les 3 étapes clés du processus se déroulant de manière anormale et ce, malgré la mise en place des meilleures conditions physico-chimiques et biologiques possibles. En effet la rapidité et la qualité de la cicatrisation d'une plaie dépendent de facteurs intrinsèques et extrinsèques. Ce processus de réparation peut donc être anormalement prolongé selon :
- l'étiologie de la plaie ;
- son état et sa localisation ;
- la survenue d'une infection causée par la présence de certains agent infectieux comme Staphylococcus *aureus* ou Pseudomonas *aeruginosa ;*
- l'existence d'une pathologie préexistante (comme le diabète, une déficience immunitaire, une insuffisance veineuse, etc...) ;
- l'environnement extérieur ; ou
- des facteurs génétiques prédisposant ou non à des troubles de la cicatrisation.

Parmi ces plaies, on retrouve les plaies chroniques telles que les ulcères veineux, les escarres ou les plaies caractéristiques des sujets diabétiques. Les plaies chroniques se définissent par une absence de cicatrisation après un délai de 6 semaines à compter de l'apparition de la plaie et ce quel que soit le traitement appliqué. Pour traiter ce type de plaies, il peut être crucial d'accélérer le processus de cicatrisation.

Les plaies du diabétique sont caractérisées comme étant un type très particulier de plaies chroniques, présentant des spécificités propres.

Le diabète est une maladie toujours plus répandue. Selon de récentes estimations, environ 285 millions de personnes dans le monde en souffrent, un chiffre qui devrait atteindre 439 millions d'individus d'ici 2030. Les diabétiques sont exposés à différentes complications liées à leur maladie. Parmi celles-ci figurent l'augmentation de l'incidence des accidents cardiovasculaires tels que les infarctus du myocarde, les AVC et des complications microvasculaires telles que la rétinopathie (pouvant aboutir à la cécité) et la néphropathie (pouvant aboutir à une insuffisance rénale). L'une des complications les plus dramatiques du diabète est l'amputation. On estime qu'à travers le monde, une personne est amputée d'un membre inférieur du fait du diabète toutes les 30 secondes et que 85 % de ces amputations sont précédées d'un ulcère du pied (Fédération Internationale du Diabète, FID 2005). Environ 15 % des personnes atteintes de diabète développeront un ulcère du pied au cours de leur vie.

L'ulcère du pied diabétique (UPD ou DFU en anglais) est défini par « une plaie profonde localisée sous la cheville chez un patient diabétique, indépendamment de sa durée » (FID, 2005). En effet, la cause première de l'absence de cicatrisation de ces plaies diabétiques est liée à une biodisponibilité du glucose exacerbée. Celle-ci induit de nombreuses modifications physiologiques et métaboliques, telles qu'un épaississement de la peau, un stress oxydatif important pouvant conduire à une neuropathie ou à une artériopathie. L'artériopathie et la neuropathie sont donc deux facteurs de risque majeurs distincts de retard de la cicatrisation des plaies diabétiques, et plus particulièrement des plaies du pied diabétique.

Les ulcères du pied du diabétique sont classés en différentes catégories. On retrouve d'un côté les ulcères du pied diabétique du patient neuropathique et d'un autre côté les ulcères du pied diabétique des patients artériopathiques qui se distinguent nettement des ulcères du pied diabétique du patient neuropathique par la présence d'une atteinte ischémique (caractérisée notamment par la diminution de l'apport sanguin artériel à un organe). Ainsi, le pied diabétique du patient neuropathique se caractérise généralement par un pied chaud, bien perfusé et un pouls pédieux palpable. De plus, le patient neuropathique présente une perte de sensibilité très nette, voire totale au niveau de sa lésion. L'ulcération est souvent localisée sur la plante du pied, sous une callosité négligée soumise à une forte pression plantaire. A l'inverse, le pied diabétique du patient artériopathique est quant à lui froid avec un pouls pédieux qui n'est pas palpable. En général, il est douloureux, car la sensibilité du patient n'est ici peu ou pas altérée. Néanmoins, ce type de patient présente une altération plus ou moins prononcée au niveau des vaisseaux de la plaie, allant d'une simple diminution de l'apport sanguin à une nécrose irréversible des différents tissus vasculaires pouvant conduire à une amputation au niveau de la zone altérée voire au-delà. Pour ce type de patient, les ulcères se situent en partie au niveau de la plante du pied, mais aussi fréquemment au bout des orteils ou dans des zones situées derrière le talon. Les différences cliniques majeures entre les formes d'ulcères du pied du diabétique imposent donc des traitements bien distincts. Pour le traitement de l'ulcère neuropathique, il est souhaitable d'éliminer le plus possible de tissus nécrotiques et de callosités afin de relancer le processus cicatriciel. A l'inverse, il convient de ne pas débrider un ulcère ischémique sous peine d'altérer la phase de néoangiogénèse préalable à la reprise du processus de cicatrisation classique. De plus, les patients atteints d'ulcères neuropathiques cicatrisent en moyenne deux à quatre fois plus rapidement que les patients atteint d'ulcère ischémique, l'ischémie entravant significativement le processus de cicatrisation et augmentaient les risques infectieux, comme il ressort par exemple de l'étude « Comparison of characteristics and healing course of diabetic foot ulcers by etiological classification : neuropathic, ischemic and neuroischemic », Totsu RR et al., J Diabetes Compplications, 2014 Jul_Aug ; 28(4) :528-35. Ainsi, les traitements proposés à ce jour pour l'ulcère du pied du diabétique chez des patients neuropathiques sont inadaptés et non transposables aux patients artériopathiques. A ce jour, pour les plaies ischémiques, aucun traitement (substitut cutané, facteur de croissance, pansement ou dispositif médical) n'a fait la preuve de son efficacité.

On reste donc à la recherche d'un traitement efficace de l'ulcère du pied du diabétique chez des patients artériopathiques dont l'ischémie complexifie significativement la guérison.

L'invention a pour objet, selon un premier aspect, un oligosaccharide polysulfaté synthétique ayant 1 à 4 unités oses, ses sels, ou ses complexes, pour son utilisation pour traiter l'ulcère du pied du diabétique chez des patients artériopathiques.

L'invention a également pour objet, selon un deuxième aspect, une composition pharmaceutique comprenant un oligosaccharide polysulfaté synthétique ayant 1 à 4 unités oses, ses sels, ou ses complexes, pour son utilisation pour traiter l'ulcère du pied du diabétique chez des patients artériopathiques.

### Patients artériopathiques

On entend par « patients artériopathiques » au sens de la présente demande, des patients diabétiques présentant une ischémie.

La distinction entre les patients neuropathiques et les patients présentant une ischémie est clairement établie par la classification du Texas décrite dans les publications « A comparison of two diabetic foot ulcer classification systems: the Wagner and the University of Texas wound classification systems ». Oyibo SO et al. , Diabetes Care. 2001 Jan;24(1): 84-8 et « A new classification of diabetic foot complications : a simple and effective teaching tool », Dr Amit Kumar C Jain et al. , The Journal of Diabetic Foot Complications, 2012; Volume 4, Issue 1, No. 1, Pages 1-5**],** qui procède à une classification en deux dimensions comme repris dans les tableaux ci-après :

| **DEGRE DE PROFONDEUR DE L'ATTEINTE** | | | |
|---|---|---|---|
| **0** | **I** | **II** | **III** |
| Lésion pré- ou post-ulcérée, totalement épithélialisée | Plaie superficielle n'atteignant pas les tendons, la capsule ou l'os | Plaie pénétrant les tendons, la capsule | Plaie pénétrant l'os ou l'articulation |

| **STADE** | **CONSTATATION CLINIQUE** |
|---|---|
| A | Pas d'infection ni d'ischémie |
| B | Infection mais pas d'ischémie |
| C | Ischémie mais pas d'infection |
| D | Infection et ischémie |

Les patients artériopathiques objets de la présente demande se trouvent dans des catégories IC et IIC alors que les patients neuropathiques se trouvent dans la classe IA.

L'ischémie peut être confirmée, en premier lieu, par la mesure de l'index de pression brachiale à la cheville inférieur ou égale à 0,9, de préférence inférieur à 0,8 et/ou la mesure de l'index de pression brachiale du gros orteil inférieur ou égale à 0,7.

L'ischémie peut encore être confirmée une pression systolique de la cheville supérieure ou égale à 70 mmHg et/ou une pression systolique du gros orteil supérieure ou égale à 50 mmHg.

La mesure de l'un ou plus de ces paramètres permet d'identifier la population de les patients artériopathies objets de l'invention.

Selon un mode particulier de réalisation, les patients artériopathiques objets de la présente demande présentent :
- un index de pression brachiale à la cheville inférieur ou égale à 0,9, de préférence inférieur à 0,8 ou la mesure de l'index de pression brachiale du gros orteil inférieur ou égale à 0,7,
   et
- une pression systolique de la cheville supérieure ou égale à 70 mmHg et/ou une pression systolique du gros orteil supérieure ou égale à 50 mmHg.

Selon un mode préféré de réalisation, les patients artériopathiques objets de la présente demande présentent :
- soit un index de pression brachiale à la cheville ≤ à 0,9, de préférence inférieur à 0,8, combiné à une pression systolique du gros orteil ≥ à 50 mmHg ou, si la mesure au gros orteil n'est pas possible (amputation), à une pression systolique de la cheville ≥ à 70 mmHg ;
- soit un index de pression brachiale à la cheville > à 0,9, combiné à une pression systolique du gros orteil ≥ à 50 mmHg et à un index de pression brachiale du gros orteil ≤ à 0,7.

### Ulcère du pied du diabétique

La présente invention propose un oligosaccharide polysulfaté synthétique ayant 1 à 4 unités oses, ses sels, ou ses complexes, pour son utilisation pour traiter l'ulcère du pied du diabétique chez des patients artériopathiques.

Comme décrit précédemment, l'ulcère du pied diabétique (UPD ou DFU en anglais) est défini par « une plaie profonde localisée sous la cheville chez un patient diabétique, indépendamment de sa durée ».

Selon un mode particulier de réalisation, l'ulcère du pied diabétique traité dans le cadre de la présente demande présente une dimension inférieure à 5 cm², c'est-à-dire que la plaie s'inscrit dans un cercle dont l'aire est de 5 cm².

Selon un mode encore préféré de réalisation, l'ulcère du pied diabétique traité dans le cadre de la présente demande n'est pas récalcitrant, c'est-à-dire qu'il s'est formé depuis moins de 6 mois.

### Oligosaccharides polysulfatés synthétiques avant 1 à 4 unités oses

Les oligosaccharides utilisés dans le cadre de la présente invention sont des oligomères synthétiques formés de 1 à 4 unités d'osés, de préférence de 1 à 3 unités d'osés, et plus préférentiellement de 1 ou 2 unités d'osés, généralement liées entre elles par liaison glycosidique alpha ou bêta. En d'autres termes, il s'agit de mono, di, tri ou tétrasaccharides, et de préférence de mono ou disaccharides.

Il n'y a pas de limitation particulière concernant la nature des unités oses de ces polysaccharides. De préférence, il s'agira de pentoses ou d'hexoses. A titre d'exemple de monosaccharide, on peut citer le glucose, le galactose ou le mannose. A titre d'exemple de disaccharide, on peut citer le maltose, le lactose, le sucrose ou le tréhalose. A titre d'exemple de trisaccharide, on peut citer le mélézitose. A titre d'exemple de tétrasaccharide, on peut citer le stachyose.

De préférence, l'oligosaccharide est un disaccharide, et de préférence encore le sucrose.

On entend par "oligosaccharide polysulfaté" au sens de la présente demande un oligosaccharide dont au moins deux, et de préférence tous les groupes hydroxyles de chaque ose ont été substitués par un groupe sulfate.

De préférence, l'oligosaccharide polysulfaté utilisé dans le cadre de la présente demande est le sucrose octasulfate.

Les oligosaccharides polysulfatés utilisés dans le cadre de la présente invention peuvent se présenter sous forme de sels ou complexes.

A titre d'exemple de sels, on peut citer les sels de métal alcalin tels que les sels de sodium, de calcium ou de potassium; les sels d'argent ; ou encore les sels d'acide aminé.

A titre d'exemple de complexes, on peut citer les complexes d'hydroxyaluminium.

Dans le cadre de la présente invention, des composés particulièrement préférés sont les suivants :
- le sel de potassium du sucrose octasulfate ;
- le sel d'argent du sucrose octasulfate ; et
- le complexe hydroxyaluminium du sucrose octasulfate, appelé communément sucralfate.

En particulier, dans le cadre de la présente invention, les oligosaccharides polysulfatés utilisés sont de préférence les sels de potassium plutôt que les sels d'aluminium du sucrose octasulfate.

Les oligosaccharides polysulfatés utilisés dans le cadre de la présente invention peuvent se présenter sous forme de poudre micronisée ou sous forme solubilisée.

Un exemple d'oligosaccharide polysulfaté utilisé dans le cadre de la présente invention est le sel de potassium du sucrose octasulfate (connu sous l'abréviation KSOS), commercialisé dans le produit Urgotul® Start par les Laboratoires URGO.

Selon un mode particulier de réalisation, l'oligosaccharide polysulfaté synthétique selon l'invention est mis en œuvre à une concentration supérieure ou égale à 70 mg/mL, de préférence supérieure ou égale à 100 mg/mL. Selon un mode préféré de réalisation, l'oligosaccharide polysulfaté synthétique selon l'invention est mis en œuvre à une concentration comprise entre 100 mg/mL et 1000 mg/mL.

### Composition

L'invention a également pour objet une composition pharmaceutique comprenant l'oligosaccharide polysulfaté synthétique précédemment décrit, pour son utilisation pour traiter l'ulcère du pied du diabétique chez des patients artériopathiques.

### Substance active additionnelle

D'une façon générale, les composés oligosaccharides selon l'invention pourront être utilisés seuls ou en mélange de deux ou plus d'entre eux, ou encore en combinaison avec une (ou plusieurs) autre(s) substance(s) active(s).

De manière générale, les actifs sont choisis parmi les anti-bactériens, les antiseptiques, les anti-douleurs, les anti-inflammatoires, les actifs favorisant la cicatrisation, les agents dépigmentants, les antiprurigineux, les filtres UV, les agents apaisants, les agents hydratants, les agents anti-oxydants, et leurs mélanges.

De manière générale, les actifs sont choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques, des sels d'argent ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, les extraits de *Centella Asiatica,* la papaïne, les silicones, les huiles essentielles de thym, de niaouli, de romarin et de sauge, l'acide hyaluronique, l'Allantoïne, - Hema'tîte (gattefossé), Vitamine C, TEGO Pep 4-17( evonik), Toniskin (silab), Collageneer (Expanscience), Timecode (Seppic), Gatuline skin repair (gattefossé), Panthenol, PhytoCellTec Alp Rose (Mibelle Biochemistry), Erasyal(libragen), Serilesine (Lipotec), Heterosides de Talapetraka (beyer), Stoechiol(codif), macarose (Sensient), Dermaveil (Ichimaru Pharcos), Phycosaccaride AI (Codif);
- les agents dépigmentants tels que l'acide kojique (Kojic Acid SL® - Quimasso (Sino Lion)), l'Arbutine (Olevatin® - Quimasso (Sino Lion)), le mélange de palmitoylpropyl de sodium et d'extrait de nénuphar blanc (Sepicalm® - Seppic), l'undécylénoyl phénylalanine (Sepiwhite® - Seppic),
- les antiprurigineux : hydrocotisone, enoxolone, diphenyhydramine, antihistaminique à application locale anti H1
- les actifs hydratants tels que xpermoist (lipotec), Acide hyaluronique, Urée, acides gras, Glycérine, Cires, Exossine(unipex)
- les filtres UV tels que Parsol MCX, Parsol 1789
- les agents apaisants tels que de la camomille, du bisabolol, du xanthalène, de l'acide glycyrrhébénique, tanactine (CPN), Calmiskin (Silab),
- les agents anti-oxydants, tels que la vitamine E.

Selon un mode préféré de réalisation, les composés oligosaccharides selon l'invention peuvent être utilisés en combinaison avec un agent anti-oxydant.

### Galénique

Les oligosaccharides polysulfatés synthétiques utilisés dans le cadre de la présente invention peuvent administrés par voie topique, et notamment mis en œuvre au sein d'une formulation galénique, comme par exemple un gel, une solution, une émulsion, une crème, des granules, des capsules de tailles variables allant du nano ou micromètre au millimètre, qui permettra leur application au niveau de la plaie. Alternativement, les composés utilisés dans le cadre de la présente invention peuvent être mis en œuvre au sein d'une solution pour injection sous-cutanée.

S'ils sont employés en mélange de deux ou plusieurs d'entre eux ou encore en combinaison avec une ou plusieurs autres substances actives, ces composés pourront être incorporés dans la même formulation galénique ou dans des formulations galéniques distinctes.

Bien entendu, la quantité d'oligosaccharides polysulfatés synthétiques selon l'invention utilisée dans la formulation galénique est adaptée en fonction de la cinétique recherchée ainsi que des contraintes spécifiques liées à sa nature, solubilité, résistance à la chaleur, etc.

### Pansement

De manière préférentielle, les composés oligosaccharides polysulfatés synthétiques utilisés dans le cadre de la présente invention, ou une formulation galénique les contenant, seront intégrés à un pansement.

Les composés oligosaccharides polysulfatés synthétiques, et notamment le sel de potassium de sucrose octasulfate ou une formulation galénique le contenant pourra être incorporé dans un élément quelconque de la structure d'un pansement sous réserve que ce composé puisse entrer directement ou indirectement en contact avec la surface de la plaie.

De préférence et afin de favoriser une action rapide, ce composé (ou une formulation galénique le contenant) sera incorporé dans la couche du pansement qui vient en contact avec la plaie ou déposé sur la surface du pansement qui vient en contact avec la plaie.

Avantageusement, le sel de potassium du sucrose octasulfate (ou une formulation galénique le contenant) pourra ainsi être déposé, de façon continue ou discontinue, sur la surface destinée à venir au contact de la plaie :
- soit sous forme liquide, par exemple par vaporisation d'une solution ou suspension le contenant ;
- soit sous forme solide, par exemple par tamisage d'une poudre le contenant.

La couche ou surface venant en contact avec la plaie pourra être constituée par exemple d'un matériau absorbant telle qu'une mousse absorbante hydrophile en polyuréthane ; un matériau textile telle qu'une compresse, comme par exemple un non tissé, un film, un voile de fibres ; un matériau adhésif absorbant ou non ; une structure interface adhérente ou non.

De façon alternative, la couche ou surface venant en contact avec la plaie pourra être constituée par exemple d'une trame textile, de préférence en polyester telle que décrite dans la demande de brevet WO 01/70285 ou dans la demande de brevet WO2013/093298 sur laquelle sera enrobée, ou enduite, une matrice élastomérique comprenant un oligosaccharide polysulfaté synthétique ayant 1 à 4 unités oses, ses sels, ou ses complexes,, en particulier un sel de potassium de sucrose octasulfate, telle que décrite dans la demande de brevet WO2008/149035 ou dans la demande WO 2014/009488.

L'invention a ainsi pour objet un pansement comprenant une trame textile enduite d'une matrice élastométique comprenant un oligosaccharide polysulfaté synthétique ayant 1 à 4 unités oses, ses sels, ou ses complexes, en particulier un sel de potassium de sucrose octasulfate, pour son utilisation pour traiter l'ulcère du pied du diabétique chez des patients artériopathiques.

De façon générale, on pourra jouer sur la galénique ou la structure du pansement pour obtenir un profil de relargage du sel de potassium de sucrose octasulfate spécifique, rapide ou retardé, selon les besoins.

Bien entendu, la quantité de sel de potassium de sucrose octasulfate utilisée dans la formulation galénique ou dans le pansement sera adaptée en fonction de la cinétique recherchée ainsi que des contraintes spécifiques liées à sa nature, solubilité, résistance à la chaleur, etc.

Par pansement, on entend désigner, au sens de la présente demande, tous types de pansements utilisés pour le traitement des plaies.

Typiquement, un pansement comprend au moins une couche ou matrice, adhésive ou non.

Les composés oligosaccharides polysulfatés synthétiques selon l'invention, ou une formulation galénique les contenant, peuvent être incorporés dans un élément quelconque de la structure d'un pansement, par exemple dans la matrice.

De préférence, et afin de favoriser une action rapide, ce composé (ou une formulation galénique le contenant) peut être incorporé dans la couche du pansement qui vient en contact avec la plaie ou déposé sur la surface de la couche du pansement qui vient en contact avec la plaie.

De telles techniques de dépôt sont bien connues de l'homme de l'art et certaines sont par exemple décrites dans la demande de brevet WO 2006/007814.

Selon une variante de l'invention, le composé oligosaccharide polysulfaté synthétique selon l'invention peut être incorporé dans un pansement absorbant à base de fibres gélifiantes, comme par exemple le produit AQUACEL® commercialisé par la société CONVATEC.

Très souvent, lors de la pose de ces pansements, le personnel soignant maintient ces derniers en place à l'aide d'une bande ou recouvre ces derniers d'un élément secondaire tel qu'un second pansement absorbant ou une bande de contention. Il est donc utile que le pansement reste fixé sur la plaie afin que le personnel soignant conserve les mains libres pour positionner ces éléments secondaires. D'une façon générale, tout type d'adhésif couramment employé dans les pansements pourra être utilisé à cet effet.

Afin de ne pas altérer les tissus sains ou les berges de la plaie, notamment lors du retrait du pansement, on préférera un adhésif ayant la propriété d'adhérer à la peau sans adhérer à la plaie.

A titre d'exemple d'un tel adhésif, on peut ainsi citer les adhésifs à base d'élastomères de silicone ou de polyuréthane, tels que les gels de silicone ou de polyuréthane, et les adhésifs hydrocolloïdes.

De tels adhésifs hydrocolloïdes sont notamment constitués d'une matrice élastomérique à base d'un ou plusieurs élastomères choisis parmi les polymères séquencés poly(styrène-oléfine- styrène) en association avec un ou plusieurs composés choisis parmi les plastifiants, tels que les huiles minérales, des résines tackifiantes et, si nécessaire, des antioxydants, dans laquelle est incorporée une quantité, de préférence faible, d'hydrocolloïdes (de 3 à 20% en poids) comme par exemple la carboxyméthylcellulose de sodium ou des polymères superabsorbants comme les produits commercialisés sous la dénomination LUQUASORB® par la société BASF.

Selon un mode préféré de réalisation, les composés oligosaccharides polysulfatés synthétiques utilisés dans le cadre de la présente invention, ou une formulation galénique les contenant, seront intégrés à un pansement comprenant un adhésif hydrocolloïde, ledit oligosaccharide polysulfaté étant incorporé dans ledit adhésif de préférence en une quantité comprise entre 1 et 15 % en poids, de préférence encore entre 5 et 10 % en poids, par rapport au poids de l'adhésif.

La formulation de tels adhésifs hydrocolloïdes est bien connue de l'homme de l'art et décrite par exemple dans les demandes de brevet FR 2 783 412, FR 2 392 076 et FR 2 495 473.

L'utilisation d'un filet d'adhésif sur le non tissé permet d'une façon particulièrement avantageuse de diminuer ou d'éviter le risque que de petites fibrilles du matériau textile viennent au contact de la plaie et s'accrochent aux tissus, en provoquant ainsi une sensation douloureuse au retrait, voire un obstacle au processus de cicatrisation de la plaie.

Selon une variante de réalisation préférée de la présente invention, le composé oligosaccharide polysulfaté synthétique selon l'invention est incorporé dans un tel adhésif à une concentration compatible avec sa solubilité et sa résistance à la chaleur.

Sur la base de ces critères, le composé oligosaccharide polysulfaté synthétique selon l'invention est utilisé de préférence en une quantité comprise entre 1 et 15% en poids, et de préférence encore entre 5 et 10% en poids, par rapport au poids total de l'adhésif.

Si l'on souhaite augmenter l'absorption de ce pansement non tissé, on pourra associer ce dernier avec une couche absorbante additionnelle, et de préférence une couche absorbante qui ne gélifie pas, comme en particulier une compresse telle que celle utilisée dans le produit URGOTUL® Duo ou URGOTUL® Trio, une mousse hydrophile absorbante, de préférence une mousse polyuréthane hydrophile présentant une capacité d'absorption supérieure à celle du non tissé telle que celle utilisée dans le produit CELLOSORB®.

Selon un mode préféré de réalisation, le composé oligosaccharide polysulfaté synthétique selon l'invention est incorporé dans un pansement non tissé, associé avec une couche absorbante additionnelle, et de préférence une couche absorbante qui ne gélifie pas, comme en particulier une compresse.

Selon un autre mode préféré de réalisation, le composé oligosaccharide polysulfaté synthétique selon l'invention est incorporé dans un pansement non tissé, associé avec une couche absorbante additionnelle, et de préférence une couche absorbante qui ne gélifie pas, comme en particulier une mousse hydrophile absorbante, de préférence une mousse polyuréthane hydrophile présentant une capacité d'absorption supérieure à celle du non tissé.

Le non tissé et la mousse peuvent être associés par des techniques bien connues de l'homme de l'art, par exemple par calandrage à chaud à l'aide d'une poudre thermofusible à base de polymères TPU/polycaprolactone.

Cette technique est couramment employée pour le liage entre eux de non tissés destinés au marché médical.

Enfin, cette mousse ou le non tissé (lorsque celui-ci est utilisé seul) peuvent être recouverts d'un support pour protéger la plaie de l'extérieur.

Ce support peut être de taille supérieure à celle des autres couches et rendu adhésif de façon continue ou discontinue sur sa face venant en contact avec la plaie afin d'optimiser le maintien du pansement lors de son usage, en particulier si la plaie se situe sur des zones corporelles non planes.

Ce support et son adhésif sont de préférence imperméables aux fluides mais très perméables à la vapeur d'eau afin de permettre une gestion optimale des exsudats absorbés par le pansement et éviter les problèmes de macération.

De tels supports sont bien connus de l'homme du métier et sont constitués par exemple de films respirants et imperméables tels que des films de polyuréthane, des complexes mousse/film ou non tissé/film.

### Additifs

Outre les agents actifs, les composés oligosaccharides selon l'invention pourront être utilisés en combinaison avec un (ou plusieurs) additifs couramment utilisés dans la préparation des pansements. Ces additifs peuvent notamment être choisis parmi les parfums, les conservateurs, les vitamines, la glycérine, l'acide citrique, etc.

L'activité des oligosaccharides polysulfatés synthétiques selon l'invention a été mise en évidence dans les exemples non limitatifs suivants.

### EXEMPLE : Mise en évidence de l'effet du sel de potassium du sucrose octasulfate (KSOS) pour traiter l'ulcère du pied du diabétique chez des patients artériopathiques.

On a conduit un essai randomisé contrôlé mené en double aveugle sur la base de 240 patients diabétiques artériopathiques répartis dans deux groupes parallèles : un groupe de contrôle de 114 patients et un groupe traité de 126 patients. Les groupes sont également répartis en sous-groupes en fonction de la dimension de la plaie et de son caractère récalcitrant. Ainsi, on distingue les patients dont les ulcères présentent une dimension inférieure à 5 cm², c'est-à-dire que la plaie s'inscrit dans un cercle dont l'aire est de 5 cm² de ceux présentant une dimension comprise entre 5 et 30 cm². On distingue également les patients dont l'ulcère traité dans le cadre de la présente demande n'est pas récalcitrant, c'est-à-dire qu'il s'est formé depuis moins de 6 mois, et les patients donc l'ulcère est récalcitrant, c'est-à-dire qu'il s'est formé depuis plus de 6 mois.

Les patients artériopathiques sont des patients présentant un diabète de type 1 ou 2 et une hémoglobine glyquée (HbA1c) ≤ 10%, et présentant un ulcère du pied du diabétique d'une surface comprise entre 1 et 30 cm².

Les patients se trouvent dans des catégories IC et IIC de la classification du Texas et présentent une ischémie caractérisée :
- Soit par un index de pression brachiale à la cheville ≤ à 0,9 combiné à une pression systolique du gros orteil ≥ à 50 mmHg ou, si la mesure au gros orteil n'est pas possible (amputation), à une pression systolique de la cheville ≥ à 70 mmHg ;
- Soit par un index de pression brachiale à la cheville > à 0,9 combiné à une pression systolique du gros orteil ≥ à 50 mmHg et à un index de pression brachiale du gros orteil ≤ à 0,7.

Après nettoyage de la plaie par une solution saline, on a appliqué au patient, soit un pansement comprenant un sel de potassium du sucrose octasulfate ou un pansement présentant la même structure mais ne contenant pas d'oligosaccharide polysulfaté.

Le pansement est constitué d'une trame textile ajourée en polyester enrobée par une matrice élastomérique, laquelle matrice est notamment décrite dans la demande de brevet WO 201009488.

Le pansement est changé tous les deux à quatre jours selon le niveau d'exsudation de la plaie et les patients sont traités pendant un maximum de 20 semaines.

Tous les mois, une évaluation clinique de la cicatrisation de la plaie est conduite sur la base des critères suivants :
- tolérance au traitement
- état de la peau périlésionnelle
- douleur de la plaie
- examen clinique de la plaie (tissus nécrotiques, tissus de granulation, escarres...)
- caractérisation de la plaie (dimension de la plaie, aspect général, analyse des berges, de la peau périlesionnelle, tracé des pourtours de la plaie pour suivre l'évolution dans le temps)

### Résultats :

Au sens du présent exemple, on entend par le terme « fermeture de plaie », toute plaie cicatrisée, c'est-à-dire jusqu'à épithélialisation complète du derme, cette dernière étant confirmée deux semaines postérieurement à la 20^{ème} semaine par des médecins spécialisés.

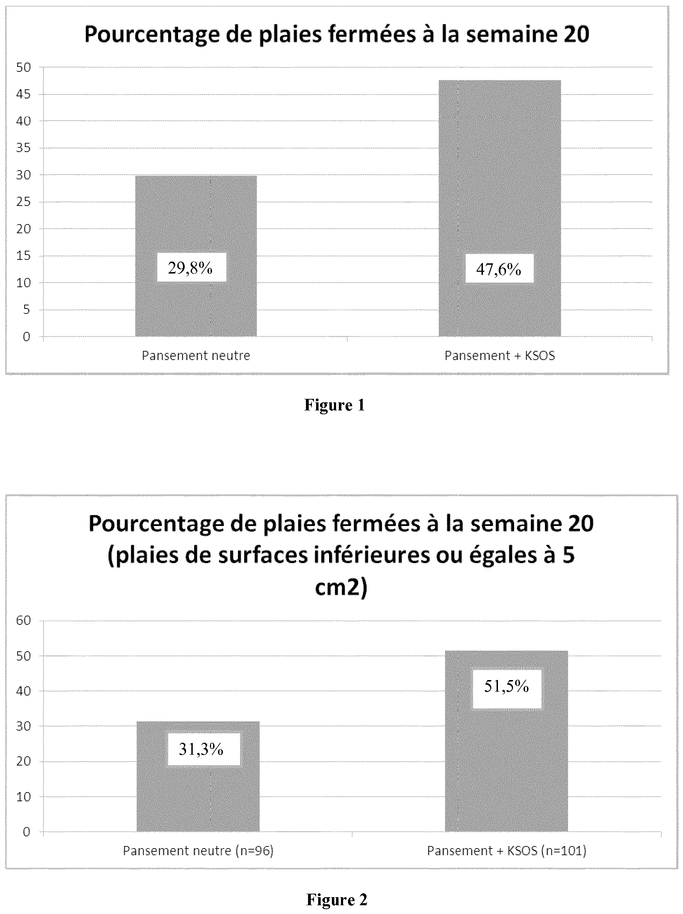

Comme illustré à la figure 1, la présente étude a mis en évidence que le plaies traitées par le pansement selon l'invention comprenant un sel de potassium du sucrose octasulfate présentent une cicatrisation significativement améliorée de l'ulcère, caractérisée notamment par un pourcentage de fermeture de plaie de 47,6% contre seulement 29,8% chez le groupe de contrôle traité avec le pansement comparatif présentant la même structure mais ne contenant pas d'oligosaccharide polysulfaté.

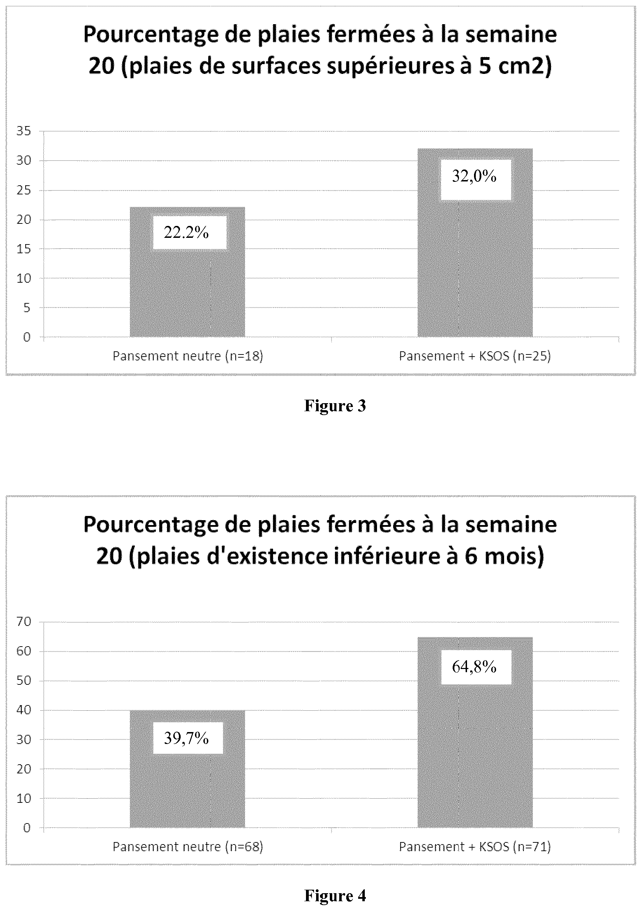

Il ressort en outre des figures 2 et 3 que lorsque les ulcères traités par le pansement selon l'invention comprenant un sel de potassium du sucrose octasulfate présentent une dimension inférieure à 5 cm², c'est-à-dire que la plaie s'inscrivent dans un cercle dont l'aire est de 5 cm², ils présentent une cicatrisation significativement améliorée, caractérisée notamment par un pourcentage de fermeture de plaie de 51,5% contre 31,3% chez le groupe de contrôle.

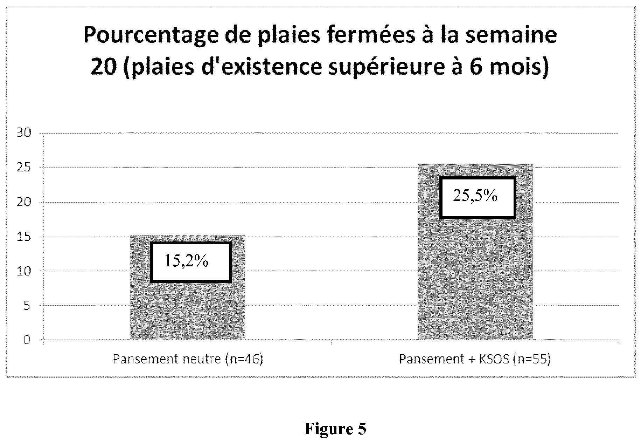

Les figures 4 et 5 montrent également que lorsque les ulcères traités par le pansement selon l'invention comprenant un sel de potassium du sucrose octasulfate ne sont pas récalcitrants, c'est-à-dire qu'ils se sont formés depuis moins de 6 mois, ils présentent une cicatrisation significativement améliorée, caractérisée notamment par un pourcentage de fermeture de plaie de 64,8% contre 39,7% chez le groupe de contrôle.

**Le tableau 1 ci-dessous croise les données des figures 2 à 5 :**

| | Groupe contrôle pansement neutre | Groupe pansement + KSOS | Différence entre le groupe pansement + KSOS et le groupe contrôle |
|---|---|---|---|
| Total | 29,8% (34/114) | 47,6% (60/126) | 17.8 |
| Plaies de surfaces ≤ 5 cm² et d'existence inférieure à 6 mois (n=116) | 42,1% (24/57) | 69,5% (41/59) | 27.4 |
| Plaies de surfaces > à 5 cm² et d'existence inférieure à 6 mois (n=23) | 27,3% (3/11) | 41,7% (5/12) | 14.4 |
| Plaies de surfaces ≤ 5 cm² et d'existence supérieure à 6 mois (n=81) | 15,4% (6/39) | 26,2% (11/42) | 10.8 |
| Plaies de surfaces > à 5 cm² et d'existence supérieure à 6 mois (n=20) | 14,3% (1/7) | 23,1% (3/13) | 8.8 |

Il ressort de ce tableau que les patients présentant un ulcère non récalcitrant (formé depuis moins de 6 mois) de dimension inférieure à 5 cm² présentent une cicatrisation particulièrement améliorée lorsqu'ils sont traités par le pansement selon l'invention comprenant un sel de potassium du sucrose octasulfate.

Il ressort du tableau 2 suivant que les patients traités par le pansement selon l'invention présentent une réduction significative de leur surface de plaie traitée (exprimée en % de réduction ou en cm² de réduction de la surface), ladite surface des plaies traitées présentant en effet une réduction médiane de 1.76 cm² pour les patients se voyant appliquer le pansement selon l'invention, contre une réduction médiane de 1,23 cm² pour les patients sur lesquels est appliqué le pansement contrôle.

**Tableau 2 :**

| | Groupe contrôle pansement neutre (n=114) | Groupe pansement + KSOS (n=126) |
|---|---|---|
| Réduction de la surface de la plaie de J0 à fin de semaine 20 (en % de surface) | -42,3 +/- 114,8 | -71,7 +/- 46,5 |
| Valeur médiane | -89,9 | -98,1 |
| Valeurs limites | -100 à 707,8 | -100 à 222,1 |
| Réduction de la surface de la plaie de J0 à fin de semaine 20 (en cm² de surface) | -2,28 +/- 5,51 | -3,17 +/- 5,22 |
| Valeur médiane | -1,23 | -1,76 |
| Valeurs limites | -28,80 à 14,44 | -32,12 à 15,53 |

Enfin, il ressort du tableau 3 suivant que le délai moyen de fermeture de plaie entre les patients traités par le pansement objet de l'invention est significativement plus rapide (119,7 +/- 4,7 jours en moyenne) que celui de groupe de patients traités par le pansement contrôle (180,5 +/- 8,7 jours en moyenne) :

**Tableau 3 :**

| | Groupe contrôle pansement neutre (n=114) | Groupe pansement + KSOS (n=126) | Différence entre le Groupe pansement + KSOS (n=126) et le groupe contrôle pansement neutre (n=114) |
|---|---|---|---|
| Délai de fermeture de plaie (en jours) | 180,5 +/- 8,7 | 119,7 +/- 4,7 | 60,8 |

Ainsi, la présente étude a permis de mettre en évidence que, quel que soit le sexe des patients traités, qu'ils présentent ou non un ulcère récalcitrant, et quelle que soit la taille de l'ulcère traité, un nombre significativement plus important de patients traités par le pansement selon l'invention comprenant un sel de potassium du sucrose octasulfate présentent une cicatrisation complète de l'ulcère par rapport aux patients traités par le pansement comparatif présentant la même structure mais ne contenant pas d'oligosaccharide polysulfaté. En outre, la cicatrisation des plaies traitées par le pansement selon l'invention est plus rapide que celle des plaies traitées par le pansement comparatif ne contenant pas d'oligosaccharide polysulfaté, ce qui est démontré par les mesures de pourcentage de fermeture de plaie réalisées. L'intégralité des résultats de la présente étude fera l'objet d'une publication sous la référence « Etude Explorer ».

Il en résulte que les oligosaccharides polysulfatés, et en particulier le sel de potassium du sucrose octasulfate permet un traitement rapide et efficace de l'ulcère du pied du diabétique chez les patients artéripathiques, en particulier présentant une ischémie.

## Revendications

1. Oligosaccharide polysulfaté synthétique ayant 1 à 4 unités oses, ses sels, ou ses complexes, pour son utilisation pour traiter l'ulcère du pied du diabétique chez des patients artériopathiques.

2. Oligosaccharide selon la revendication 1, pour son utilisation pour activer la cicatrisation de l'ulcère du pied du diabétique chez des patients artériopathiques.

3. Oligosaccharide pour son utilisation selon la revendication 1 ou 2, **caractérisé en ce que** sa concentration est supérieure ou égale à 70 mg/mL, de préférence 100 mg/mL, et plus préférentiellement comprise entre 100 et 1000 mg/mL.

4. Oligosaccharide pour son utilisation selon l'une quelconque des revendications précédentes, **caractérisé en ce qu'**il comprend 1 à 3 unités oses, 1 ou 2 unités oses choisies de préférence parmi les pentoses et les hexoses, ainsi que les sels et complexes de ces composés.

5. Oligosaccharide pour son utilisation selon l'une quelconque des revendications précédentes, **caractérisé en ce qu'**il est choisi parmi :
- le sel de potassium du sucrose octasulfate ;
- le sel d'argent du sucrose octasulfate ; et
- le complexe hydroxyaluminium du sucrose octasulfate.

6. Oligosaccharide pour son utilisation selon l'une quelconque des revendications précédentes **caractérisé en ce que** ce soit le sel de potassium de sucrose octasulfate.

7. Oligosaccharide pour son utilisation selon l'une quelconque des revendications précédentes, **caractérisé en ce qu'**il est mis en œuvre sous la forme d'une composition telle qu'un gel, une solution, une émulsion, une crème, des granules ou des capsules permettant une application directement au niveau de la plaie.

8. Composition pharmaceutique comprenant un oligosaccharide polysulfaté synthétique ayant 1 à 4 unités oses, ses sels, ou ses complexes, pour son utilisation pour traiter l'ulcère du pied du diabétique chez des patients artériopathiques.

9. Pansement comprenant un oligosaccharide polysulfaté synthétique ayant 1 à 4 unités oses, ses sels, ou ses complexes, en particulier un sel de potassium de sucrose octasulfate, pour son utilisation pour traiter l'ulcère du pied du diabétique chez des patients artériopathiques.

10. Pansement pour son utilisation selon la revendication 9, comprenant une trame textile enduite d'une matrice élastométique, ladite matrice comprenant ledit oligosaccharide polysulfaté synthétique.

## Patentansprüche

1. Synthetisches polysulfatiertes Oligosaccharid mit 1 bis 4 Zucker-Einheiten, Salze davon oder Komplexe davon, zur Verwendung bei der Behandlung von diabetischem Fußulkus bei arteriopathischen Patienten.

2. Oligosaccharid nach Anspruch 1 zur Verwendung bei der Förderung der Heilung eines diabetischen Fußulkus bei arteriopathischen Patienten.

3. Oligosaccharid zur Verwendung nach Anspruch 1 oder 2, **dadurch gekennzeichnet, dass** seine Konzentration größer oder gleich 70 mg/mL, vorzugsweise 100 mg/mL und weiter bevorzugt zwischen 100 und 1000 mg/mL ist.

4. Oligosaccharid zur Verwendung nach einem der vorhergehenden Ansprüche, **dadurch gekennzeichnet, dass** es 1 bis 3 Zucker-Einheiten, vorzugsweise 1 oder 2 aus Pentosen und Hexosen ausgewählte Zucker-Einheiten, sowie Salze und Komplexe dieser Verbindungen umfasst.

5. Oligosaccharid zur Verwendung nach einem der vorangehenden Ansprüche, **dadurch gekennzeichnet, dass** es ausgewählt ist aus:
- dem Kaliumsalz von Saccharose-Oktasulfat;
- dem Silbersalz von Saccharose-Oktasulfat und
- dem Hydroxyaluminiumkomplex von Saccharose-Oktasulfat.

6. Oligosaccharid zur Verwendung nach einem der vorangehenden Ansprüche, **dadurch gekennzeichnet, dass** es das Kaliumsalz von Saccharose-Oktasulfat ist.

7. Oligosaccharid zur Verwendung nach einem der vorhergehenden Ansprüche, **dadurch gekennzeichnet, dass** es in Form einer Zusammensetzung, wie einem Gel, einer Lösung, einer Emulsion, einer Creme, Granulat oder Kapseln ausgeführt ist, die eine direkte Anwendung auf die Wunde ermöglicht.

8. Pharmazeutische Zusammensetzung, umfassend ein synthetisches polysulfatiertes Oligosaccharid mit 1 bis 4 Zucker-Einheiten, Salze davon oder Komplexe davon, zur Verwendung bei der Behandlung von diabetischem Fußulkus bei arteriopathischen Patienten.

9. Verband, umfassend ein synthetisches polysulfatiertes Oligosaccharid mit 1 bis 4 Zucker-Einheiten, Salze davon oder Komplexe davon, insbesondere ein Kaliumsalz von Saccharose-Oktasulfat, zur Verwendung bei der Behandlung von diabetischem Fußulkus bei arteriopathischen Patienten.

10. Verband zur Verwendung nach Anspruch 9, umfassend einen Textilschuss, der mit einer elastomeren Matrix beschichtet ist, wobei die Matrix das synthetische polysulfatierte Oligosaccharid umfasst.

## Claims

1. Synthetic polysulphated oligosaccharide having 1 to 4 monosaccharide units, salts thereof, or complexes thereof, for use thereof for treating diabetic foot ulcer in arteriopathic patients.

2. Oligosaccharide according to claim 1, for use thereof for activating the healing of diabetic foot ulcer in arteriopathic patients.

3. Oligosaccharide for use thereof according to claim 1 or 2, **characterised in that** the concentration thereof is greater than or equal to 70 mg/mL, preferably 100 mg/mL, and more preferentially between 100 and 1000 mg/mL.

4. Oligosaccharide for use thereof according to any one of the preceding claims, **characterised in that** it comprises 1 to 3 monosaccharide units, 1 or 2 monosaccharide units chosen preferably from pentoses and hexoses, as well as the salts and complexes of these compounds.

5. Oligosaccharide for use thereof according to any one of the preceding claims, **characterised in that** it is chosen from:
- potassium sucrose octasulfate salt;
- silver sucrose octasulfate salt; and
- hydroxyaluminium sucrose octasulfate complex.

6. Oligosaccharide for use thereof according to any one of the preceding claims **characterised in that** it is potassium sucrose octasulfate salt.

7. Oligosaccharide for use thereof according to any one of the preceding claims, **characterised in that** it is used in the form of a composition such as a gel, a solution, an emulsion, a cream, granules or capsules enabling application directly on the wound.

8. Pharmaceutical composition comprising a synthetic polysulphated oligosaccharide having 1 to 4 monosaccharide units, salts thereof, or complexes thereof, for use thereof for treating diabetic foot ulcer in arteriopathic patients.

9. Dressing comprising a synthetic polysulphated oligosaccharide having 1 to 4 monosaccharide units, salts thereof, or complexes thereof, in particular a potassium sucrose octasulfate salt, for use thereof for treating diabetic foot ulcer in arteriopathic patients.

10. Dressing for use thereof according to claim 9, comprising a textile weft coated with an elastomeric matrix comprising said synthetic polysulphated oligosaccharide.
